# EUROPEAN PATENT SPECIFICATION

(11) **EP 3 641 881 B1**
(45) Date of publication and mention of the grant of the patent: **28.07.2021**
(21) Application number: 18729858.3
(22) Date of filing: 21.05.2018
(51) Int. Cl.: A61N 5/06

(54) **CURATIVE CAPSULE**
HEILKAPSEL
CAPSULE DE TRAITEMENT

(30) Priority: 12.06.2017 BG 11252317
(43) Date of publication of application: 29.04.2020
(73) Proprietor: ICDSOFT LTD, 1421 Sofia (BG)
(72) Inventor: DIMITROV, Dimitar Stoev, 1408 Sofia (BG)
(74) Representative: Ilarionov, Pavko Jordanov
(86) International application number: PCT/BG2018/000023
(87) International publication number: WO 2018/227260

(56) References cited:
- WO-A1-2008/136958
- US-A1- 2013 053 928

## Description

### FIELD OF THE INVENTION

The invention relates to a curative capsule to be applied in the field of medicine and more particularly for elimination of *Helicobacter Pylory* from the human stomach.

### BACKGROUND OF THE INVENTION

It is known that *Helicobacter Pylory* causes chronic gastritis, ulcer or even gastric cancer.

It is believed that more than 50% of the world population, especially in the developing countries, is having the bacteria. The way of spreading of the bacteria is not fully clear, but it is presently believed that the infection spreads through contaminated water and food supplies.

Different combinations of antibiotics are used for eradication of *Helicobacter Pylory* but this kind of treatment has serious unwanted side effects. These antibiotics are also relatively expensive. In addition, there are antibiotic resistant strains.

It is also known that *Helicobacter Pylory* is very sensitive to ultraviolet (UV) light and perish quickly when treated with such light.

A device for eradicating of *Helicobacter Pylory* from a patient's stomach using UV radiation is known from DE 10 2010 010 763 A1. The known device consists of a probe, the outer tube of which is inserted in the patient's esophagus. In the outer tube are placed two smaller tubes, in one of which is placed a fiber bundle conducting UV radiation to the stomach from an outer source of radiation (located outside of the patient's body). The second tube is connected to a source for fluid delivery (water solution or air) heated to 37° C, which temperature is being measured by a sensor mounted on the outer surface of the outer tube. Before starting of the UV radiation, a stopper is inserted in the patient's stomach and located on the pillory. The aim of introducing of fluid is to inflate the stomach so as the epithelium fringes to open and the UV radiation to reach unobstructed each fold of the stomach. During the manipulation the patient is lying down on a table mounted on an axis which allowed periodical inclination of the patient's body.

The use of such probe is causing significant discomfort and therefore the manipulation with the probe is performed after the placement of the patient under anesthesia. After such an intervention, the patient must stay in the hospital for a certain period of time.

This manipulation is expensive, requires a lot of time, specialized equipment and a highly qualified team of specialists.

The multiple use of such probe requires a thorough cleaning after every intervention and there is a risk of transmitting the infection from patient to patient.

A healing capsule (US 2013/053928) (D1), with a UV emitter connected to an energy source is also known. The UV emitter and the energy source are housed inside the shell of the healing capsule. The shell of the healing capsule is made of UV-permeable material, which is insoluble in gastric juices.

The known therapeutic capsule has balloons of spongy material that can be expanded at a given pH to slow down the movement of the capsule.

Alternatively, the capsule can be moved through the digestive system by magnetic force. Once arriving at its destination, the capsule releases a detachable head, which anchors into the digestive tract using anchors. The anchors can be composed of gelatin or another biodegradable polymer.

The moving of the healing capsule using magnetic force is complicated and difficult to be made at home. Furthermore, the design proposed in D1, including a splittable capsule is a complicated solution and would require a professional medical supervision.

The document WO 2008/136958 (D2) also discloses one or more phototherapeutic capsules introduced into the gastrointestinal tract, swallowed or otherwise inserted. The capsule(s) contains a battery, a UVA1 -LED, an activation triggering mechanism means, a computer chip to store, data, a central processor unit (CPU), any suitable communication means and a pH monitor. The activated state, when the capsule will deliver the phototherapeutic prescription, is controlled by the activation trigger means (D2, p 80). The capsule can be manipulated using magnetic force (p 81, second paragraph - p 82, first paragraph).

The system disclosed in D2 is a very complex system, requiring a constant control of a medical professional.

### SUMMARY OF THE INVENTION

The task of the invention is to create a curative capsule with simplified construction for single use by means of which to be achieved a painless eradication of Helicobacter Pylori from the patient's stomach, which can also be performed at home without the necessity of specialized equipment and highly qualified team of specialists as well as without causing discomfort to the patient.

This task is solved by curative capsule containing a source of energy connected to an UV emitter.

According to the invention, the UV emitter and the source of energy are connected to each other and placed within the shell of the curative capsule.

The shell of the curative capsule is made of UV radiation permeable material, insoluble in gastric juices.

According to one embodiment of the invention, the curative capsule in addition is encapsulated in a clear gelatin layer where the outer surface of that layer is rugged with a plurality of conical sections.

The diameter of the sphere-shaped curative capsule without the gelatin shell is less than or equal to 6 mm, whereas the outer diameter of the curative capsule with the gelatin layer is not smaller than 12 mm.

The advantages of the invention are:
- the treatment is painless;
- does not cause discomfort to the patient;
- the need of sedation falls away;
- no need for specialized equipment and team of specialists;
- the manipulation can be done at home;
- the curative capsules are for single use by one patient;
- the treatment is many times cheaper and accessible to a wide range of patients.

### BRIEF DSCRIPTION OF THE DRAWINGS

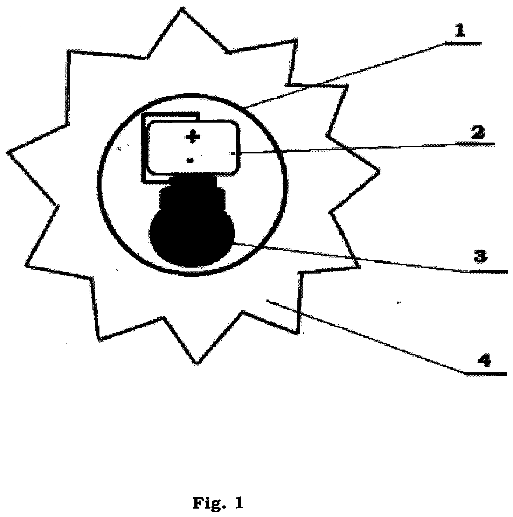

Fig. 1 shows the curative capsule according to the invention in cross section.

### DETAILED DESCRIPTION OF THE INVENTION

The curative capsule according to the invention consists of a shell 1 within which is placed an energy source 2 (a miniature battery) connected to an UV emitter 3 - Fig. 1.

The shell 1 of the curative capsule is made of a UV radiation permeable material and insoluble in gastric juices and other liquids, food or non-food products which may be found in the stomach of the patient.

The source of energy 2 is connected with the UV emitter 3, whereby the source of energy 2 and the UV emitter 3 are place in the shell 1.

The shell 1 of the curative capsule itself is covered by a rugged gelatin layer 4 with a plurality of conical sections (horns). The rugged gelatin layer 4 is also permeable to UV radiation.

By means of the gelatin layer 4 are achieved two effects:
- The initial size of the capsule is increased to such an extend that the capsule can not pass into the pylori prior to the degradation of the gelatin, respectively prior to the end of the therapeutic treatment of the epithelium by the UV rays;
- The cones (horns) of the gelatin layer 4 penetrate between the epithelium fringes of the stomach wall and thus allow the UV light to penetrate therein. This eliminates the need for closing the pylorus and fluid swelling of the stomach.

The shell 1 together with the rugged gelatin layer 4, is usually in the form of a sphere with a diameter not smaller than 12mm, whereby the shell 1 without the gelatin layer is around 6mm.

12mm is a size that can be swallowed by the patient without tension, but at the same time large enough not to go through the pylorus.

However, a 6-millimeter curative capsule can easily pass through the patient's pylorus after the manipulation and leave the gastrointestinal tract of the patient after achieving the desired healing effect.

### USE OF THE INVENTION

The curative capsule, when swallowed by the patient, passes through the esophagus and falls into his stomach. Due to its size, the capsule, while wrapped with the rugged gelatin layer 4 (12mm in diameter), can not pass through the pylorus.

The healing capsule begins to move in the stomach, the horns of the rugged gelatin layer 4 penetrate between the fringes of the stomach epithelium and thus allow the UV rays to reach each fold of the stomach and destroy Helicobacter Pylori.

After about 30 minutes the gelatin layer 4 is already dissolved by the gastric juices, the diameter of the curative capsule has reached 6mm and the healing capsule passes freely through the pylorus of the patient. At the same time, the energy of the energy source 2 is spent and the UV emitter 3 ceases to function.

## Claims

1. A curative capsule with a rounded shape, having a UV emitter (3) connected to a source of energy (2), both placed inside the curative capsule, whereby the shell (1) of the curative capsule is made of UV rays permeable material that is insoluble in gastric juices,
**characterized in that,**
the shell (1) is enveloped in a rugged gelatin layer (4), which is UV rays permeable and soluble in gastric juices and its outer surface is formed with a plurality of conical sections.

2. A curative capsule according to claim 1, **characterized in that,** the diameter of the sphere-shaped curative capsule without the gelatin layer (4) is less than or equal to 6 mm.

3. A curative capsule according to claims 1 and 2, **characterized in that,** the outer diameter of the curative capsule with the gelatin layer (4) is not smaller than 12 mm.

## Patentansprüche

1. **Heilkapsel** mit abgerundeter Form, ausgestattet mit einem UV-Strahler (3), der mit einer Energiequelle (2) verbunden ist, die sich innerhalb der Heilungskapsel befinden, wobei die Hülle (1) der Heilkapsel aus einem UV-durchlässigen, magensaftunlöslichen Material besteht,
**dadurch gekennzeichnet, dass**
die Hülle (1) der Heilungskapsel von einer rauen Gelatineschicht (4) umgeben ist, löslich in Magensaft, deren äußere Oberfläche von mehreren konischen Abschnitten gebildet wird.

2. **Heilkapsel,** gem. Anspruch 1, **dadurch gekennzeichnet, dass** der Durchmesser der kugelförmigen Heilkapsel ohne Gelatineschicht (4) kleiner oder gleich 6 mm ist.

3. **Heilkapsel,** gem. Anspruch 1 und 2, **dadurch gekennzeichnet, dass** der Außendurchmesser der Heilkapsel mit der Gelatineschicht (4) nicht kleiner als 12 mm ist.

## Revendications

1. **Gélule de traitement** de forme arrondie, munie d'un émetteur UV (3), relié à une source d'énergie (2), qui sont logés à l'intérieur de la gélule de traitement, l'enveloppe (1) de la gélule de traitement étant faite d'une matière transmettant les rayons UV, ui est insoluble dans les sucs gastriques,
**caractérisée en ce que**
l'enveloppe (1) de la gélule de traitement recouverte d'une couche de gélatine rugueuse (4), soluble par les sucs gastriques, ont la surface extérieure est formée par une multitude de sections coniques.

2. **Gélule de traitement,** selon la revendication 1, **caractérisée en ce que** le diamètre de la gélule de traitement de forme sphérique sans la couche de gélatine (4) est inférieur ou égal à 6 mm.

3. **Gélule de traitement,** selon les revendications 1 et 2, **caractérisée en ce que** le diamètre extérieur de la gélule de traitement recouverte de la couche de gélatine (4) n'est pas inférieur à 12 mm.
